(19) Europäisches Patentamt
European Patent Office
Office européen des brevets

(11) Veröffentlichungsnummer: **0 185 080**
**B1**

(12) **EUROPÄISCHE PATENTSCHRIFT**

(45) Veröffentlichungstag der Patentschrift: **08.11.89**

(51) Int. Cl.⁴: **C 07 C 50/02**, C 07 C 57/34, C 07 C 55/28, C 07 C 57/42, C 07 C 59/84, C 07 C 69/618, C 07 C 69/608, A 61 K 31/20

(21) Anmeldenummer: **85903247.6**

(22) Anmeldetag: **15.06.85**

(86) Internationale Anmeldenummer:
**PCT/EP 85/00288**

(87) Internationale Veröffentlichungsnummer:
**WO 86/00298 (16.01.86** Gazette **86/2)**

# (54) ALPHA-, OMEGA-DICARBONSÄUREN, VERFAHREN ZU IHRER HERSTELLUNG UND ARZNEIMITTEL, DIE DIESE VERBINDUNGEN ENTHALTEN.

(30) Priorität: **22.06.84 DE 3423166**

(43) Veröffentlichungstag der Anmeldung:
**25.06.86 Patentblatt 86/26**

(45) Bekanntmachung des Hinweises auf die Patenterteilung:
**08.11.89 Patentblatt 89/45**

(84) Benannte Vertragsstaaten:
**AT BE CH DE FR GB IT LI LU NL SE**

(56) Entgegenhaltungen:
**EP-A- 0 081 930**
**DE-A- 1 210 786**
**DE-A- 2 644 789**

(73) Patentinhaber: **YISSUM RESEARCH DEVELOPMENT COMPANY OF THE HEBREW UNIVERSITY OF JERUSALEM, 46, Jabotinsky Street P.O. Box 4279, Jerusalem 91042 (IL)**

(72) Erfinder: **BAR-TANA, Jacob, 14 Harav Shrim Street, Jerusalem (IL)**
Erfinder: **WITTE, Ernst-Christian, Beethovenstrasse 2, D-6800 Mannheim 1 (DE)**
Erfinder: **HAGENBRUCH, Bernd, In den Gärten 5, D-6840 Lampertheim (DE)**
Erfinder: **PILL, Johannes, In der Keitgasse 6, D-6906 Leimen 3 (DE)**
Erfinder: **STEGMEIER, Karlheinz, Kirchbergstrasse 17, D-6148 Heppenheim (DE)**

(74) Vertreter: **Whalley, Kevin et al, MARKS & CLERK 57/60 Lincoln's Inn Fields, London WC2A 3LS (GB)**

**EP 0 185 080 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

## Beschreibung

In der EP-A1-0 081 930 sind einige langkettige α,ω-Dicarbonsäuren der allgemeinen Formel I

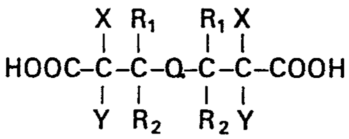
(I)

sowie in-vivo hydrolysierbare Derivate der Carbonsäuregruppen beschrieben, in der

$R_1$ und $R_2$ jeweils einen unsubstituierten oder substituierten Kohlenwasserstoff-Rest oder Heterocyclus,

X und Y jeweils Wasserstoff, gegebenenfalls substituiertes Alkyl, Halogen, Cyano, Carboxyl, niederes Alkoxycarbonyl oder Carbamoyl, und

Q ein Diradikal, das aus einer linearen Kette mit 8 bis 14 C-Atomen besteht, die durch inerte Substituenten substituiert sein kann oder einer oder mehrere dieser C-Atome bzw. Heteroatome gegebenenfalls einen Ring bilden können, bedeuten.

Diese Verbindungen finden Verwendung als Arzneimittel zur Behandlung von Fettleibigkeit, Hyperlipidämie oder Diabetes.

Beispielhaft offenbart sind in jener Patentanmeldung lediglich Verbindungen der Formel I, in denen X Wasserstoff, Ethoxycarbonyl, Brom, Cyano oder Methyl, Y Wasserstoff, $R_1$ und $R_2$ jeweils Methyl und Q eine $-(CH_2)_8-$, $-(CH_2)_{10}-$ oder $-(CH_2)_{12}$-Gruppe bedeuten.

Es wurde nun gefunden, dass Verbindungen der Formel I mit einer durch einen Cyclohexyliden- oder einen Phenylen-Ring unterbrochenen Kette Q, die formal unter die breite Definition der Formel I der EP-A1-0 081 930 fallen, eine ausgeprägte lipidsenkende Wirkung aufweisen. Zusätzlich zeigen sie eine antidiabetische Wirkung.

Gegenstand der vorliegenden Erfindung sind daher langkettige, α,ω-Dicarbonsäuren der allgemeinen Formel I'

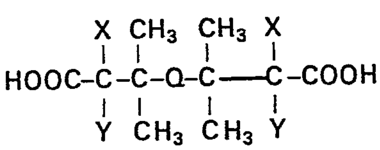
(I')

in der

X und Y die gleich oder verschieden sein können, Wasserstoff, $C_1$–$C_6$-Alkyl, $C_1$–$C_6$-Alkoxy, Hydroxy, Cyano, Halogen, Carboxyl, $C_1$–$C_6$-Alkoxycarbonyl oder Carbamoyl, und

Q eine $-(CH_2)_n$-Cyclohexyliden-$(CH_2)_n$, eine $-(CH_2)_m$-Phenylen-$(CH_2)_m$, eine $-CH_2-CH=CH-$ Phenylen-$CH=CH-CH_2-$ oder eine $-CH_2-CH=CH-CH_2$ -Phenylen- $CH_2-CH=CH-CH_2$-Gruppe darstellen und

n die Zahlen 2, 3 oder 4 und

m 3 oder 4 bedeuten, sowie deren in-vivo hydrolisierbare Carbonsäure-Derivate.

Eine analoge Verbindung 1,4-Phenylen-bis-(3.3-dimethyl-5-yl-pentansäure) ist von A.T. Blomquist et al. [Am. Soc. 80 (1958) 3405] als Zwischenprodukt beschrieben ohne Angabe einer pharmakologischen Wirksamkeit.

Unter Alkyl der Substituenten X und Y sind Gruppen mit 1–6, insbesondere 1–4, C-Atomen zu verstehen. Bevorzugt ist der Methyl- und Ethyl-Rest.

Unter Alkoxy der Substituenten X und Y sind Gruppen mit 1–6, insbesondere 1–4, C-Atomen zu verstehen. Bevorzugt ist der Methoxy- und Ethoxy-Rest.

Alkoxycarbonyl-Gruppen der Substituenten X und Y sind Gruppen mit 1–6 C-Atomen. Bevorzugt ist der Methoxycarbonyl- und Ethoxycarbonyl-Rest.

Unter Halogen sind in allen Fällen Fluor, Chlor und Brom zu verstehen.

Das Phenyl- sowie das Cyclohexyl-Ringsystem der Kette Q kann über die 1,2-, 1,3- oder 1,4-Stellung in die Kette eingebaut sein.

Bevorzugt sind Verbindungen der Formel I', in denen X und Y die folgenden Bedeutungen haben:

X und Y, gleich oder verschieden, Wasserstoff, Halogen, Methoxy, Hydroxy, Ethoxycarbonyl, Cyano, Carbamoyl oder Carboxyl, insbesondere jedoch X und Y jeweils Wasserstoff oder Y Wasserstoff und X Halogen, Ethoxycarbonyl, Hydroxy, Methoxy, Cyano, Carbamoyl oder Carboxyl.

In-vivo hydrolysierbare Derivate der Verbindungen der Formel I' sind z.B. Salze mit pharmazeutisch verträglichen Alkali-, Erdalkali- oder Ammonium-Basen; Ester, insbesondere niedere Alkylester wie Methyl-, Ethyl- und Isopropylester; Amide; mono- oder dialkylierte Amide wie Dimethylamide; oder Lactone, die mit einem OH-Substituenten der Formel I' gebildet werden können.

Gegenstand der Erfindung sind auch cis-/trans-Isomere der ungesättigten Verbindungen.

Die Herstellung der Verbindungen der Formel I' geschieht in an sich bekannter Weise, in dem man

a) eine Dihalogenid der Formel II

Hal–Q–Hal (II),

in der

Hal Chlor oder Brom bedeuten und

Q die angegebene Bedeutung hat, in eine Bis-Grignard-Verbindung überführt und diese mit 2 Mol einer Verbindung der Formel III

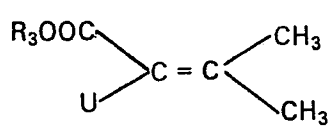
(III),

in der

$R_3$ eine niedere Alkylgruppe und

U eine $COOR_3$-Gruppe oder eine $-CONH_2$- oder CN-Gruppe darstellt, umsetzt, die erhaltenen Verbindungen gewünschtenfalls verseift, gewünschtenfalls decarboxyliert und gewünschtenfalls in α und ω-Stellung die Substituenten Alkyl, Alkoxy, Hydroxy oder Halogen einführt oder

b) für den Fall, dass X und Y Wasserstoff, Halogen, Hydroxy, Alkyl oder Alkoxy bedeuten, eine Bis-triphenylphosphonium-Verbindung der Formel IV

$$\overset{\ominus}{Z}(Ph)_3\overset{\oplus}{P}-Q_1-\overset{\oplus}{P}(Ph)_3\overset{\ominus}{Z} \quad (IV)$$

in der

Z Chlorid oder Bromid und

$Q_1$ eine $-(CH_2)_p$-Phenylen-$(CH_2)_p$- oder $-(CH_2)_p$-Cyclohexyliden-$(CH_2)_p$-Gruppe darstellt, in der p die Zahlen 1 oder 2 sein können, bedeuten mit 2 Mol eines Carbonsäureesters der Formel V

$$R_3OOC{-}CH_2{-}\overset{\displaystyle CH_3}{\underset{\displaystyle CH_3}{C}}{-}Q_2{-}CH{=}O \quad (V),$$

in der

$Q_2$ Valenz oder eine $-CH_2$-Gruppe und

$R_3$ eine niedere Alkylgruppe darstellen, umsetzt, die erhaltenen ungesättigten Verbindungen hydriert, gewünschtenfalls in α- und ω-Stellung die Substituenten Alkyl, Alkoxy, Halogen oder Hydroxy einführt, sowie gewünschtenfalls anschliessend die erhaltenen Ester, Amide oder Salze in freie Säuren oder die freien Säuren in Salze, Ester oder Amide überführt.

Nach dem Verfahren a) werden in der Regel Tetracarbonsäure-Ester erhalten. Diese Verbindungen können durch Alkali verseift werden und durch Erhitzen zu Dicarbonsäuren decarboxyliert werden. Die erhaltenen Verbindungen mit X, Y = Wasserstoff können dann in α und ω-Stellung halogeniert werden. Dies geschieht in der Regel durch Fluor, Brom oder Chlor sowie durch entsprechende N-Succinimide. Es können aber auch α und ω-Dichlor-Verbindungen mit geeigneten Fluorierungsmittel wie Tetrabutylammoniumfluorid zu den α und ω-Difluor-Verbindungen umgesetzt werden. Die Einführung einer Alkylgruppe geschieht mit entsprechenden Alkylhalogeniden nach Umsetzung z.B. mit n-Butyllithium.

Hydroxygruppen in α- und ω-Stellung können z.B. aus entsprechenden Dichlor-Verbindungen durch alkalische Verseifung eingeführt werden.

Alkoxygruppen können durch Umsetzung der entsprechenden Dichlor-Verbindungen mit Alkoholaten (z.B. Natriummethyl) in α- und ω-Stellung eingeführt werden.

Nach Verfahren a) können auch Dicarbonsäureester anfallen, die α- und ω-Stellung einen Nitril- oder Carbamoyl-Substituenten tragen, wobei anschliessend der Nitril-Rest in eine Carbamoyl- und weiter zu einer Carboxyl-Gruppe verseift werden kann oder gegebenenfalls eine Carbamoyl-Gruppe zu einer Nitril-Gruppe nach üblichen Methoden dehydratisiert werden kann.

Nach Verfahren b) erhält man Verbindungen mit X und Y = Wasserstoff. Hierzu werden die entsprechenden Bis-phosphoniumsalze der Formel IV mit 2 Mol eines Carbonsäureesters der Formel V in Gegenwart von starken Basen wie z.B. KOH oder Natriummethylat zur Umsetzung gebracht, wobei ein Diester mit mindestens zweifach ungesättigtem Rest Q entsteht. Der Rest $Q_1$ kann analog Q substituiert, unterbrochen oder Bestandteil eines Ringsystems sein.

Anstelle der Bis-phosphoniumsalze der Formel IV lassen sich auch entsprechende Phosphinoxide bzw. Phosphonester (P-O-aktivierte Olefinbildungsreaktion) einsetzen.

Die nach Verfahren b) erhaltenen ungesättigten Verbindungen können anschliessend zu den gesättigten Verbindungen hydriert werden. Ebenfalls können Phenylen-Verbindungen zu Cyclohexyliden-Verbindungen hydriert werden.

Die Phenylen- oder Cycloalkyliden-Verbindungen können auch hergestellt werden, indem man eine Verbindung der Formel IV

$$Me[2-(CH_2)_q-A-(CH_2)_q-Me]2 \quad (VI),$$

in welcher

Me ein zweiwertiges Metall wie z.B. Cadmium oder Zink darstellt,

A Phenylen oder Cyclohexyliden und

q die Zahl 1 oder 2

bedeuten, mit einer Verbindung der Formel VII

$$T-(CH_2)_q-\overset{\displaystyle CH_3}{\underset{\displaystyle CH_3}{C}}-CH_2COOAlk \quad (VII),$$

in welcher

T eine aktive Carbonsäure-Funktion, insbesondere eine Säurehalogenid-oder eine Säureanhydrid-Gruppe darstellt und

q und Alk die oben angegebene Bedeutung haben, umsetzt, die so erhaltenen Diketodicarbonsäureester in üblicher Weise verseift und anschliessend gegebenenfalls die Ketogruppen reduziert.

Für die Olefinbildung haben sich als starke Basen Alkalimetall-alkoholate wie Lithium- oder Natriummethylat, Alkaliamide wie Natriumamid, aber auch Lithium-Organyle wie z.B. Butyllithium, sowie Natriumhydrid bewährt. Die Reaktionen werden bevorzugt in niederen Alkoholen oder in Ethern wie Diethylether oder Tetrahydrofuran durchgeführt.

Die Hydrierung der nach Verfahren b) resultierenden Bis-alkene erfolgt unter den für diese Reaktion üblichen Bedingungen in Gegenwart von Metallkatalysatoren wie z.B. Palladium auf Kohle bei Normaldruck, bevorzugt aber unter erhöhtem Druck und erhöhter Temperatur.

Zur Hydrierung des Phenylenringes erwiesen sich Platin, Rhodium oder Ruthenium als besonders geeignete Katalysatoren.

Die nachträgliche Einführung der Substituenten X und Y in die α- und ω-Stellung geschieht nach den gleichen Methoden wie unter Verfahren a) beschrieben.

Zur Reduktion der Ketogruppen der nach Verfahren a) erhaltenen Diketosäuren eignet sich das Verfahren nach Huang-Minlon (Erhitzen eines Gemisches aus Keton, wässrigem Alkali, Glykol und Hydrazin), oder man reduziert mit Zink bzw. kupferhaltigem Zink und Salzsäure (Clemmensen-Reduktion); man kann aber auch in das entsprechende Tosylhydrazon überführen und dieses mit komplexen Borhydriden reduzieren.

Die Veresterung der Säure gemäss Formel I' geschieht in üblicher Weise über das entsprechende Säurechlorid. Hierzu wird die freie Säure mit Thionylchlorid in das Säurechlorid überführt und dieses mit dem entsprechenden Alkohol umgesetzt.

Die Verseifung der anfallenden Ester gemäss Formel I' geschieht üblicherweise mit Alkali in alkoholischer Lösung. Sie kann auch in sauren Medien mit einem Gemisch aus konzentrierter Schwefelsäure und Oleum durchgeführt werden.

Als pharmakologisch verträgliche Salze kommen insbesondere Alkali-, Erdalkali- und Ammoniumsalze in Frage.

Die Salze erhält man in üblicher Weise z.B. durch Neutralisation der Verbindungen der Formel I' mit den entsprechenden Laugen oder Säuren.

Zur Herstellung von Arzneimitteln werden die Verbindungen der allgemeinen Formel I' in an sich bekannter Weise mit geeigneten pharmazeutischen Trägersubstanzen, Aroma-, Geschmacks- und Farbstoffen gemischt und beispielsweise als Tabletten oder Dragees ausgeformt oder unter Zugabe entsprechender Hilfsstoffe in Wasser oder Öl, wie z.B. Olivenöl, suspendiert oder gelöst.

Die Substanzen der allgemeinen Formel I' können in flüssiger oder fester Form oral und parenteral appliziert werden. Als Injektionsmedium kommt vorzugsweise Wasser zur Anwendung, welches die bei Injektionslösungen üblichen Stabilisierungsmittel, Lösungsvermittler und/oder Puffer enthält. Derartige Zusätze sind z.B. Tartrat- oder Borat-Puffer, Ethanol, Dimethylsulfoxid, Komplexbildner (wie Ethylendiamintetraessigsäure), hochmolekulare Polymere (wie flüssiges Polyethylenoxid) zur Viskositätsregulierung oder Polyethylen-Derivate von Sorbitanhydriden.

Feste Trägerstoffe sind z.B. Stärke, Lactose, Mannit, Methylcellulose, Talkum, hochdisperse Kieselsäure, höhermolekulare Polymere (wie Polyethylenglykole).

Für die orale Applikation geeignete Zubereitungen können gewünschtenfalls Geschmacks- und Süssstoffe enthalten. Für die äusserliche Anwendung können die erfindungsgemässen Substanzen I' auch in Form von Pudern und Salben verwendet werden. Sie werden dazu z.B. mit pulverförmigen, physiologisch verträglichen Verdünnungsmitteln bzw. üblichen Salbengrundlagen vermischt.

Die verabreichte Dosis hängt vom Alter, der Gesundheit und dem Gewicht des Empfängers, dem Ausmass der Krankheit, der Art gleichzeitiger gegebenenfalls durchgeführter weiterer Behandlungen, der Häufigkeit der Behandlungen und der Art der gewünschten Wirkung ab. Üblicherweise beträgt die tägliche Dosis der aktiven Verbindung 0.1 bis 50 mg/kg Körpergewicht. Normalerweise sind 0.5 bis 40 und vorzugsweise 1.0 bis 20 mg/kg/Tag in einer oder mehreren Anwendungen pro Tag wirksam, um die gewünschten Resultate zu erhalten.

Versuchsbericht

Stellvertretend für die neuen Verbindungen wurde für die Verbindungen der Beispiele 3 und 6 b) die lipidsenkende Wirkung bestimmt und mit der besten Verbindung der EP-OS 81 930, der 3.3.14.14-Tetramethyl-hexadecan- 1.16-disäure (M 16) verglichen.

Hierzu wurde jeweils 10 männlichen Sprague-Dawley Ratten die zu testende Substanz 29 Tage lang in einer Dosis von 50 mg/kg/d und 500 mg/kg/d in Methylcellulose-Suspension verabreicht. Am Versuchsende, 3 Stunden vor der letzten Sondierung, wurden die Cholesterin- und Triglyceridwerte im Serum bestimmt.

In der nachstehenden Tabelle sind die Veränderungen in % im Vergleich zu Kontrollkollektiven angegeben.

| Verbindung | mg/kg/d | Cholesterin-Senkung (%) | Triglycerid-Senkung (%) |
|---|---|---|---|
| Bsp. 3 | 50 | 31 | 51 |
| | 500 | 58 | 70 |
| Bsp. 6b) | 50 | 42 | 51 |
| | 500 | 62 | 66 |
| M 16 | 50 | 24 | 42 |
| | 500 | 38 | 55 |

Bevorzugt im Sinne der Erfindung sind insbesondere die nachfolgend aufgeführten Verbindungen:

1) 1.2-Cyclohexyliden-bis-(3.3-dimethyl-7-yl)-heptansäure)

2) 1.2-Phenylen-bis-(3.3)- dimethyl-7-yl-heptansäure)

In den nachfolgenden Beispielen ist die Herstellung einiger erfindungsgemässer Verbindungen näher erläutert:

Beispiel 1

1.4- Phenylen-bis- [(1.1- dimethyl-but-4-yl)-propandisäurediethylester]

Man tropft eine aus 3,3 g Magnesiumspänen, 20,0 g (62,5 mmol) 1,4-Bis-(3-brompropyl)-benzol und 150 ml abs. Tetrahydrofuran hergestellte Grignard-Lösung zu einer −20 °C kalten Lösung aus 25,6 g (124 mmol) Isopropyliden malonsäure-diethylester und hält anschliessend 2–3 Stunden auf Rückflusstemperatur. Der abgekühlte Ansatz wird auf angesäuertes Eiswasser gegossen. Man trennt die wässrige Phase ab, extrahiert sie zweimal mit Ether, wäscht die vereinigten organischen Phasen mit Sodalösung, trocknet mit $Na_2SO_4$ und dampft ein. Der Eindampfrückstand wird bei 0,01 bar auf ca. 150 °C erhitzt, um flüchtige Nebenprodukte zu entfernen, danach bleiben 23,6 g (67% d.Th.) eines viskosen Öles zurück. Die Reinigung einer kleinen Menge mittels HPLC ergab ein viskoses Öl, das auf DC-Fertigplatten Merck KG 60/F 254 einen Rf-Wert von 0,8 (n-Heptan-Essigester 1+1) bzw. 0,5 (n-Heptan-Essigester 2+1) hat.

NMR (DMSO): δ = 1,02 (s, 12H); 1,12 (tr, 12H); 1,40 (m, 4H); 3,30 (s, 2H); 4,07 (qu, 8H); 7,03 (m, 4H).

Beispiel 2

1,4- Phenylen-bis- [(1,1- dimethyl-buty-4-yl)-propandisäure]

Ein Gemisch aus 2,5 g (4,4 mmol) des Tetraethylesters aus Beispiel 1, 25 ml Methanol und 1,0 g (25 mmol) Natriumhydroxid wird 60 Stunden auf Rückflusstemperatur gehalten, dann kühlt man ab, fügt etwas Wasser zu und extrahiert mit Ether. Danach säuert man an, wobei die Säure zunächst als Öl ausfällt. Nach dem Kristallisieren saugt man sie ab, wäscht mit Wasser und trocknet. Ausbeute 1,8 g (90% d.Th.), Schmelzpunkt 181–183 °C (Zers.)

NMR (DDMSO): δ = 1,03 (s, 12H); 1,40–1,60 (m, 8H); 2,48 (m, 4H); 3,12 (s, 2H); 7,07 (s, 4H).

Beispiel 3

1,4-Phenylen-bis- (3,3-dimethyl-6-yl- hexansäure) erhält man durch 2-stündiges Erhitzen von 1,4- Phenylen his- [(1,1- dimethyl-but-4-yl)-propandisäure] (Beispiel 2) unter Stickstoff auf 160 °C.

Ausbeute 31% d.Th., Schmelzpunkt 119–121 °C (Cyclohexan)

NMR (DDMSO) δ = 0,93 (s, 12H); 1,30 (m, 4H); 1,53 (m, 4H); 2,08 (s, 4H); 2,49 (t, 4H); 7,07 (4H).

Beispiel 4

1,4-Phenylen-bis-(3,3-dimethyl-6-yl-5-hexansäure-methylester)

Zu einem Gemisch aus 6,3 g (40 mmol) 3,3-Dimethyl- 5-oxo-pentansäure- methylester, 60 ml absolutes Methanol und 14,0 g (20 mmol) 1,4-Phenylen-bis-(methyltriphenyl- phosphoniumchlorid) tropft man bei Raumtemperatur eine aus 0,92 g (40 mgAtom) Natrium und 50 ml absolutem Methanol gebildete Natriummethylatlösung, rührt weitere drei Stunden bei Raumtemperatur und dampft dann ein. Man löst den Rückstand in Methylenchlorid, filtriert und dampft wieder ein. Nach Säulenchromatographie (zur Beseitigung einer geringen Menge fluoreszierenden Materials) mittels $CH_2Cl_2$/Kieselgel erhält man 3,8 g (49% d.Th.) eines farblosen Öles.

Isomerengemisch.

NMR ($CDCl_3$): δ = 1,02 (12H); 2,00–2,55 (8H); 3,58 und 3,63 (6H); 5,23–6,83 (4H); 7,27 (4H).

In Analogie dazu erhält man

a) 1,3-Phenylen-bis-(3,3-dimethyl-6-yl-5-hexensäure-methylester)

aus 3,3-Dimethyl-5-oxo-pentansäure-methylester und 1,3-Phenylen-bis- (methyltriphenylphosphoniumchlorid).

Ausbeute 60% d.Th. öliges Produkt.

Isomeren-Gemisch

NMR ($CDCl_3$): δ = 1,05 (12H); 2,15–2,50 (8H); 3,58 und 3,67 (6H); 5,25–6,73 (4H); 7,23 (4H).

Beispiel 5

1,4-Phenylen-bis-(3,3-dimethyl-6-yl-hexansäure-methylester)

Ein Gemisch aus 2,0 g 1,4-Phenylen-bis-(3,3-dimethyl-6-yl-5-hexensäure-methylester), Beispiel 4, 50 ml Ethanol und einer Spatelspitze 10% Pd auf Kohle-Katalysator wird in der Schüttelapparatur bis zur Beendigung der Wasserstoffaufnahme bei Normaldruck hydriert. Nach Absaugen des Katalysators dampft man ein und erhält 1,5 g (74% d.Th.) farbloses Öl.

NMR ($CDCl_3$): δ = 0,97 (s, 12H); 1,17–1,78 (m, 8H); 2,17 (s, 4H); 2,37–2,70 (m, 4H); 3,60 (s, 4H); 7,07 (s, 4H).

In Analogie dazu erhält man

a) 1,3-Phenylen-bis-(3,3-dimethyl-6-yl-hexansäure-methylester) durch Hydrieren von 1,3-Phenylen-bis-(3,3-dimethyl-6-yl- 5-hexensäure-methylester), Beispiel 4 a).

Ausbeute 94% d.Th., farbloses Öl.

NMR ($CDCl_3$): δ = 0,98 (s, 12H); 1,10–1,93 (m, 8H); 2,20 (s, 4H); 2,38–2,73 (m, 4H); 3,63 (6H); 6,83–7,23 (m, 4H).

Beispiel 6

1,4-Phenylen- bis- (3,3-dimethyl- 6-yl-hexansäure)

Man hält ein Gemisch aus 0,5 g des Methylesters (Beispiel 5), 5 ml Methanol und 5 ml 2 N-NaOH drei Stunden auf 90 °C, destilliert das Methanol ab, fügt Wasser zu und extrahiert mit Ether. Dann wird die wässrige Phase angesäuert, mit Ether extrahiert und der Etherextrakt mit $Na_2SO_4$ getrocknet. Nach dem Eindampfen erhält man 0,4 g (86% d.Th.) Produkt mit dem Schmelzpunkt 120–121 °C (Cyclohexan). Das Produkt ist mit dem nach Beispiel 3 erhaltenen identisch.

In Analogie dazu erhält man

a) 1,3-Phenylen-bis-(3,3-dimethyl-6-yl-hexansäure)

durch Verseifen ihres Methylesters (Beispiel 5a).

Ausbeute 91% d.Th., farbloses Öl.

NMR (DDMSO): δ = 0,93 (s, 12H); 1,30 (m, 4H); 1,53 (m, 4H); 2,07 (s, 4H); 2,49 (t, 4H); 6,92–7,00 (m, 3H); 7,15 (t, 1H).

b) 1,4-Phenylen-bis-(3,3-dimethyl-6-yl-5-hexensäure) durch Verseifen ihres Methylesters (Beispiel 4).

Ausbeute 67% d.Th., Schmp. 149–151 °C (Aceton) Isomerengemisch

NMR (DDMSO): δ = 1,00 (s, 12H); 2,10 (s, 4H); 2,38 (m, 4H); 5,47–5,92 (m, 2H); 6,24–6,57 (m, 2H); 7,27 (m, 4H).

c) 1,3-Phenylen-bis-(3,3-dimethyl-6-yl-5-hexensäure) durch Verseifen ihres Methylesters (Beispiel 4 a).

Ausbeute 91% d.Th., Öl, $N_D^{20}$ = 1,5433 Isomerengemisch.

NMR (DDMSO): δ = 1,00 (s, 12H); 2,13 (s, 4H); 2,22–2,50 (m, 4H), 5,5–5,95 (m, 2H); 6,22–6,65 (m, 2H); 7,18 (m, 4H).

Beispiel 7

1,4-Cyclohexyliden-bis-(3,3-dimethyl-6-yl- hexansäuremethylester)

Man hydriert in Gegenwart von Ruthenium-IVoxid bei 90 °C und 80 bar ein Gemisch aus 0,7 g 1,4-Phenylen-bis- (3,3-dimethyl-6- yl-hexansäure-methylester), Beispiel 5, und 50 ml Methanol, filtriert und dampft ein.

Ausbeute 0,7 g (98% d.Th.) farbloses Öl.

NMR ($CDCl_3$): δ = 0,97 (s, 12H); 1,10–1,50 (m, 22H); 2,18 (s, 4H); 3,65 (6H).

In Analogie dazu erhält man

a) 1,3-Cyclohexyliden-bis-(3,3-dimethyl-6-yl-hexansäuremethylester) aus dem entsprechenden 1,3-Phenylenanalogon.

Ausbeute 69% d.Th. eines farblosen Öles.

NMR ($CDCl_3$): δ = 0,98 (s, 12H); 1,07–1,88 (m, 22H); 2,20 (s, 4H); 3,65 (s, 6H).

Beispiel 8

1,4-Cyclohexyliden-bis-(3,3-dimethyl-6-yl-hexansäure)

durch Verseifen des Methylesters (Beispiel 7) in Analogie zu Beispiel 6.

Ausbeute 76% d.Th., Schmelzpunkt 167–169 °C (Essigester).

NMR (DDMSO): δ = 0,94 (s, 12H); 1,06–1,76 (m, 22H); 2,07 (s, 4H) und in gleicher Weise

a) 1,3-Cyclohexyliden-bis-(3,3-dimethyl-6-yl-hexansäure) aus Verbindung des Beispiels 7a).

Ausbeute 89% d.Th., Schmelzpunkt 74–76 °C (Essigester).

NMR (DDMSO): δ = 0,94 (s, 12H); 1,06–1,76 (m, 22H); 2,06 (s, 4H).

Beispiel 9

1,4-Phenylen-bis-(3,3-dimethyl-7-yl- 5-heptensäure)

a) 1,4-Phenylen-bis-(ethyltriphenyl-phosphonium-bromid)

Man erhitzt eine Mischung aus 5,84 g (20,0 mmol) 1,4-Bis-(2-bromethyl)-benzol und 13,1 g (50,0 mmol) Triphenylphosphin unter $N_2$-Atmosphäre 15 min auf 220 °C, dann 30 min auf 250 °C.

Das nach dem Abkühlen erstarrte Rohprodukt wird aus Ethanol umkristallisiert: 6,6 g (40%) farblose Kristalle vom Schmelzpunkt 262–263 °C.

b) 1,4-Phenylen-bis-(3,3-dimethyl-7-yl)-5-heptensäure)

Zu einer gerührten Suspension von 8,17 g (10,0 mmol) 1,4-Phenylen-bis-(ethyltriphenylphosphonium-bromid) in 300 ml wasserfreiem Ether gibt man bei Raumtemperatur unter $N_2$-Atmosphäre 21 ml einer 1,2 M Lösung von n-Butyllithium in Hexan, rührt 15 min nach, tropft dann eine Lösung von 2,65 g (20,0 mmol) 3,3-Dimethyl-5-oxo-pentansäure-methylester in 10 ml Ether zu, und erhitzt anschliessend 2 Stunden unter Rückfluss.

Nach dem Abkühlen wird der Niederschlag abgesaugt, das Filtrat eingeengt, der ölige Rückstand in 40 ml 1 N KOH und 10 ml Ethanol aufgenommen und 2 Stunden auf 50 °C erwärmt. Danach engt man auf die Hälfte ein, extrahiert mehrmals mit Dichlormethan, säuert die wässrige Phase mit 2 N HCl an, und extrahiert diese mehrmals mit Dichlormethan. Das nach Trocknen und Einengen der organischen Phase erhaltene Öl wird mit Ligroin zur Kristallisation gebracht: 1,0 g (26%) farblose Kristalle vom Schmelzpunkt 78–80 °C.

NMR (DDMSO): δ = 0,99 (s, 12H); 2,13 (s, 4H); 2,17 (d, J=7,2Hz; 4H); 3,33 (d, J=6,9Hz; 4H); 5,50–5,64 (m, 4H) 7,08 (s, 4H).

Beispiel 10

1,3-Phenylen-bis-(3,3-dimethyl-7-yl-5-heptensäure)

a) 1,3-Phenylen-bis-(ethyltriphenyl-phosphonium-bromid)

erhält man in Analogie zu Beispiel 9 a) aus 1,3-Bis-(2-bromethyl)-benzol und Triphenylphosphin.

Ausbeute 42% d.Th., farblose Kristalle, Schmelzpunkt 219–220 °C (Methanol).

b) 1,3-Phenylen-bis- (3,3-dimethyl- 7-yl-5-heptensäure) in Analogie zu Beispiel 9b) unter Verwendung von 1,3-Phenylen-bis-(ethyltriphenyl)-phosphonium-bromid).

Ausbeute 51% d.Th., farbloses Öl, $n_D^{26}$ = 1,5202.

Rf = 0,55 (DC-Fertigplatten Merck KG 60/Toluol-Dioxan-Essigsäure 90:25:10) bzw. 0,27 (n-Heptan-Essigester 1:1)

Beispiel 11

1,4-Phenylen-bis-(3,3-dimethyl-7-yl-heptansäure) wird aus 1,4-Phenylen-bis-(3,3-dimethyl-7-yl-5-heptensäure), Beispiel 9, durch Normaldruckhydrierung mit Palladium als Katalysator erhalten.

Ausbeute 52% d.Th., Schmelzpunkt 117-119 °C.

NMR ($CDCl_3$): δ = 1,00 (s, 12H); 1,27–1,47 (m, 12H); 2,20 (s, 4H); 2,47–2,73 (m, 4H); 7,08 (s, 4H).

Analog dazu erhält man

a) 1,3-Phenylen-bis-(3,3-dimethyl-7-yl-heptansäure)

Ausbeute 41% d.Th. farblose Kristalle, Schmelzpunkt 63–64 °C.

NMR ($CDCl_3$): δ = 1,00 (s, 12H); 1,27–1,80 (m, 12H); 2,22 (s, 4H); 2,43–2,77 (m, 4H); 6,87–7,20 (m, 4H).

Beispiel 12

Durch Hydrieren der entsprechenden 1,4-Phenylen-bis-säure bzw. der 1,3-Phenylen-bis-säure am Rhodium-Kontakt erhält man in Analogie zu Beispiel 7:

a) 1,4-Cyclohexyliden-bis-(3,3-dimethyl-7-yl-heptansäure)

Ausbeute: 72% d.Th., farbloses Öl.

NMR ($CDCl_3$): δ = 0,77–1,85 (m, 26H); 1,02 (s, 12); 2,22 (s, 4H).

b) 1,3-Cyclohexyliden-bis-(3,3-dimethyl-7-yl-heptansäure)

Ausbeute: 66% d.Th.

Schmelzpunkt 52–55 °C (Wasser).

NMR ($CDCl_3$): δ = 0,80–1,80 (m, 26H); 1,02 (s, 12H); 2,23 (s, 4H).

Beispiel 13

1,4-Phenylen -bis- (3,3-dimethyl- 5-oxo- 7-yl-heptansäure)

Zu 2,40 g (0,10 g-Atom) Magnesium-Spänen tropft man unter Rühren 14,6 g (50,0 mmol) 1,4-Bis-(2-bromethyl)-benzol in 100 ml wasserfreien Ether so zu, dass das Reaktionsgemisch siedet. Nach beendeter Zugabe refluxiert man 1,5 Stunden, kühlt ab und trägt rasch 10,1 g (55,0 mmol) Cadmiumchlorid ein. Man refluxiert erneut für 45 min, destilliert den Ether ab und gibt 100 ml Benzol zum Reaktionsgemisch. Die so erhaltene Suspension wird rasch und unter heftigem Rühren in eine Lösung von 17,3 g (50,0 mmol) 3,3-Dimethylglutarsäure-methylesterchlorid in 25 ml Benzol gegeben, 45 min refluxiert, gekühlt und durch Zugabe von 2 N $H_2SO_4$ zersetzt. Das nach Abtrennen, Trocknen und Einengen der organischen Phase erhaltene Rohprodukt wird in 100 ml Ethanol und 100 ml 1 N KOH gelöst und 3 Stunden auf 60 °C erhitzt.

Anschliessend engt man auf die Hälfte ein, extrahiert mehrfach mit Ether und säuert mit 2 N HCl an. Nun extrahiert man die wässrige Phase mehrmals mit Ether, trocknet die vereinigten Extrakte und engt ein. Das zurückbleibende Öl wird durch Verreiben mit Ligroin/Ether zur Kristallisation gebracht: 4,1 g (20%) farblose Kristalle vom Schmelzpunkt 116–120 °C (Isopropanol).

NMR ($CDCl_3$): δ = 1,06 (s, 12H); 2,55 (s, breit; 8H); 2,50–3,00 (m, 8H); 7,03 (s, 4H).

Beispiel 14

1,4-Phenylen-bis-(3,3-dimethyl-7-yl- heptansäure)

Man erhitzt eine Mischung aus 1,05 g (2,50 mmol) 1,4-Phenylen-bis-(3,3-dimethyl-5-oxo- 7-yl-heptansäure), Beispiel 13, 1,00 g (17,0 mmol) Kaliumhydroxid und 1,03 g (20,0 mmol) Hydrazinhydrat in 10 ml Diethylenglykol 2 Stunden unter Rückfluss, dann 5 Stunden auf 200 °C, wobei man das Wasser abdestilliert. Anschliessend kühlt man ab, giesst in 70 ml Wasser und säuert mit verdünnter Salzsäure an. Man rührt 1 Stunde nach und sammelt den sich abscheidenden Niederschlag:

0,60 g (61%) farblose Kristalle vom Schmelzpunkt 118–120 °C (Toluol).

Die Verbindung ist identisch mit der nach Beispiel 11 erhaltenen.

Zur Herstellung der Titelverbindung tropft man eine eiskalte Mischung aus gleichen Gewichtsteilen konzentrierter Schwefelsäure und Oleum unter äusserem Kühlen zu pulverisiertem Diester, rührt bei Eisbadtemperatur 10 Minuten lang und gibt eventuell weiteres Schwefelsäure-Oleum-Gemisch zu, so dass der Diester vollständig gelöst wird. Das Reaktionsgemisch wird dann auf −78 °C abgekühlt und eine Mischung von Eis und Methylenchlorid zugegeben. Nach dem Schmelzen des Eises trennt man die Schichten, extrahiert die wässrige Phase mit Methylenchlorid und trocknet die vereinten organischen Phasen mit Natriumsulfat. Eindampfen des Lösungsmittels liefert die Titelverbindung in einer Ausbeute von 78% d.Th., Schmp. 154–154,5 °C (aus Cyclohexan).

## Patentansprüche

1. α, ω-Dicarbonsäuren der allgemeinen Formel I'

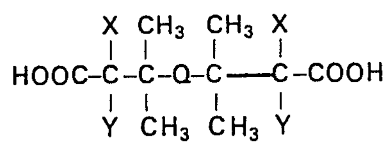

(I')

in der

X und Y die gleich oder verschieden sein können, Wasserstoff, $C_1$–$C_6$-Alkyl, $C_1$–$C_6$-Alkoxy, Hydroxy, Cyano, Halogen, Carboxyl, $C_1$–$C_6$-Alkoxycarbonyl oder Carbamoyl, und

Q eine $-(CH_2)_n$-Cyclohexyliden-$(CH_2)_n$, eine $-(CH_2)_m$-Phenylen-$(CH_2)_m$, eine $-CH_2-CH=CH$-Phenylen-$CH=CH-CH_2-$ oder eine $-CH_2-CH=CH-CH_2$-Phenylen-$CH_2-CH=CH-CH_2$-Gruppe darstellen und

n die Zahlen 2, 3 oder 4 und

m 3 oder 4 bedeuten, sowie deren in-vivo hydrolisierbare Carbonsäure-Derivate.

2. Verbindungen gemäss Anspruch 1, in denen Y Wasserstoff und X Wasserstoff, Halogen, Methoxy, Hydroxy, Cyano, Ethoxycarbonyl, Carbamoyl oder Carboxyl und Q die angegebene Bedeutung hat.

3. Verbindungen gemäss Anspruch 1 oder 2, in dem X und Y jeweils Wasserstoff und Q die angegebene Bedeutung hat.

4. 1–4-Phenylen-bis- (3,3-dimethyl- 6-yl-hexansäure) und 1,4-Phenylen-bis-(3,3-dimethyl-6-yl-5-hexensäure) nach einem der Ansprüche 1, 2 oder 3.

5. Verbindungen gemäss einem der Ansprüche 1 bis 4, in denen in-vivo hydrolysierbare Carbonsäure-Derivate, Salze mit pharmakologisch verträglichen Alkali-, Erdalkali- oder Ammoniumbasen, Ester mit niedrigen Alkoholen, Amide mit Ammoniak oder Niederalkylaminen oder gegebenenfalls Lactone darstellen.

6. Verfahren zur Herstellung von α,ω-Dicarbonsäuren der allgemeinen Formel I'

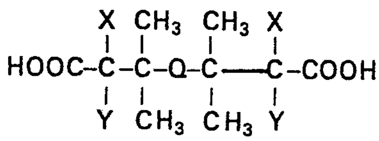
(I')

in der

X und Y die gleich oder verschieden sein können, Wasserstoff, $C_1$–$C_6$-Alkyl, $C_1$–$C_6$-Alkoxy, Hydroxy, Cyano, Halogen, Carboxyl, $C_1$–$C_6$-Alkoxycarbonyl oder Carbamoyl, und

Q eine $-(CH_2)_n$-Cyclohexyliden-$(CH_2)_n$, eine $-(CH_2)_m$-Phenylen-$(CH_2)_m$, eine $-CH_2-CH=CH$-Phenylen-$CH=CH-CH_2-$ oder eine $-CH_2-CH=CH-CH_2$-Phenylen-$CH_2-CH=CH-CH_2$-Gruppe darstellen und

n die Zahlen 2, 3 oder 4 und

m 3 oder 4 bedeuten, sowie deren in-vivo hydrolisierbare Carbonsäure-Derivate, dadurch gekennzeichnet, dass man in an sich bekannter Weise

a) ein Dihalogenid der Formel II

Hal–Q–Hal (II),

in der

Hal Chlor oder Brom bedeuten und

Q die angegebene Bedeutung hat, in eine Bis-Grignard-Verbindung überführt und diese mit 2 Mol einer Verbindung der Formel III

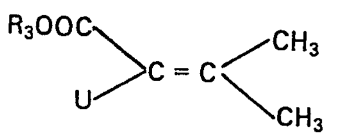
(III),

in der

$R_3$ eine niedere Alkylgruppe und

U eine $COOR_3$-Gruppe oder eine $-CONH_2-$ oder CN-Gruppe darstellt, umsetzt, die erhaltenen Verbindungen gewünschtenfalls verseift, gewünschtenfalls decarboxyliert und gewünschtenfalls in α- und ω-Stellung die Substituenten Alkyl, Alkoxy, Hydroxy oder Halogen einführt, oder

b) für den Fall, dass X und Y Wasserstoff, Halogen, Hydroxy, Alkyl oder Alkoxy bedeuten, eine Bis-triphenylphosphonium-Verbindung der Formel IV

$$\overset{\ominus}{Z}(Ph)_3\overset{\oplus}{P}-Q_1-\overset{\oplus}{P}(Ph)_3\overset{\ominus}{Z} \quad (IV),$$

in der

Z Chlorid oder Bromid und

$Q_1$ eine $-(CH_2)_p$-Phenylen-$(CH_2)_p$- oder $-(CH_2)_p$-Cyclohexyliden-$(CH_2)_p$-Gruppe darstellt, in der p die Zahl 1 oder 2 sein können, bedeuten mit 2 Mol eines Carbonsäureesters der Formel V

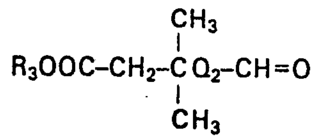
(V),

in der

$Q_2$ Valenz oder eine $-CH_2$-Gruppe und

$R_3$ eine niedere Alkylgruppe darstellen, umsetzt, die erhaltenen ungesättigten Verbindungen hydriert, gewünschtenfalls in α- und ω-Stellung die Substituenten Alkyl, Alkoxy, Halogen oder Hydroxy einführt, sowie gewünschtenfalls anschliessend die erhaltenen Ester, Amide oder Salze in freie Säuren oder die freien Säuren in Salze, Ester oder Amide überführt.

7. Arzneimittel gekennzeichnet durch einen Gehalt aus Verbindungen gemäss einem der Ansprüche 1–5 und übliche Träger- und Hilfsstoffe.

8. Verwendung der Verbindungen gemäss einem der Ansprüche 1–5 zur Herstellung von Arzneimitteln mit lipidsenkender und antidiabetischer Wirkung.

## Revendications

1. Acides α,ω-dicarboxyliques de formule générale I':

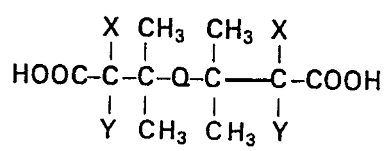
(I')

dans laquelle

X et Y, qui peuvent être identiques ou différents, représentent chacun un atome d'hydrogène, un groupe alkyle en $C_1$ à $C_6$, alcoxy en $C_1$ à $C_6$, hydroxyle, cyano, halogéno, carboxyle, alcoxy (en $C_1$ à $C_6$)-carbonyle ou carbamoyle, et

Q représente un groupe $-(CH_2)_n$-cyclohexylidène-$(CH_2)_n$, un groupe $-(CH_2)_m$-phénylène-$(CH_2)_m$, un groupe $-CH_2-CH=CH$-phénylène-$CH=CH-CH_2-$ ou un groupe $-CH_2-CH=CH-CH_2$-phénylène-$CH_2-CH=CH-CH_2-$, et

n est un nombre valant 2, 3 ou 4, et

m est un nombre valant 3 ou 4, ainsi que leurs dérivés de type acide carboxylique, hydrolysables «in vivo».

2. Composés selon la revendication 1, dans lesquels Y représente un atome d'hydrogène et X représente un atome d'hydrogène ou d'halogène, un groupe méthoxy, hydroxyle, cyano, éthoxycarbonyle, carbamoyle ou carboxyle, et Q a le sens indiqué.

3. Composés selon la revendication 1 ou 2, dans lesquels X et Y représentent chacun un atome d'hydrogène et Q a le sens indiqué.

4. Acide 1,4-phénylène-bis-(3,3-diméthyl-6-yl-hexanoïque) et acide 1,4-phénylène-bis-(3,3-diméthyl-6-yl- 5-hexenoïque) selon l'une des revendications 1, 2 ou 3.

5. Composés selon l'une des revendications 1 à 4, dans lesquels des dérivés d'acides carboxyliques hydrolysables «in vivo» représentent des sels formés avec des bases alcalines, alcalino-terreuses ou d'ammonium, physiologiquement tolérables, des esters avec des alcools inférieurs, des amides avec l'ammoniaque ou avec des alkylamines inférieures ou éventuellement des lactones.

6. Procédé pour préparer des acides α, ω-dicarboxyliques de formule générale I':

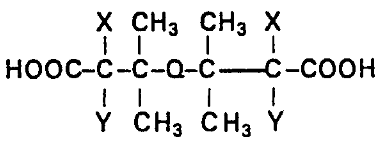

(I')

dans laquelle

X et Y, qui peuvent être identiques ou différents, représentent chacun un atome d'hydrogène, un groupe alkyle en $C_1$ à $C_6$, alcoxy en $C_1$ à $C_6$, hydroxyle, cyano, halogéno, carboxyle, alcoxy (en $C_1$ à $C_6$)-carbonyle ou carbamoyle, et

Q représente un groupe $-(CH_2)_n$-cyclohexylidène-$(CH_2)_n$, un groupe $-(CH_2)_m$-phénylène-$(CH_2)_m$, un groupe $-CH_2-CH=CH$-phénylène-$CH=CH-CH_2-$ ou un groupe $-CH_2-CH=CH-CH_2$-phénylène-$CH_2-CH=CH-CH_2-$, et

n est un nombre valant 2, 3 ou 4, et

m est un nombre valant 3 ou 4, ainsi que leurs dérivés de type carboxylique, hydrolysables «in vivo», procédé caractérisé en ce que, de façon connue en soi,

a) On transforme un dihalogénure de formule II

Hal–Q–Hal (II)

dans laquelle

Hal représente du chlore ou du brome, et

Q a le sens indiqué, en un composé de bis-Grignard et l'on fait réagir celui-ci avec 2 moles d'un composé de formule III

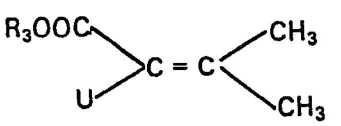

(III)

dans laquelle

$R_3$ est un groupe alkyl inférieur et

U représente un groupe-$COOR_3$ ou un groupe $-CONH_2$ ou un groupe –CN, on saponifie éventuellement les composés obtenus, on les décarboxyle éventuellement et éventuellement on introduit en position α et en position ω les substituants alkyles, alcoxy, hydroxyles ou halogéno, ou

b) si X et Y représentent chacun un atome d'hydrogène ou d'halogène ou un groupe hydroxyle, alkyle ou on fait réagir le composé de bis-triphénylphosphonium de formule IV

$$\overset{\ominus}{Z}(Ph)_3\overset{\oplus}{P}-Q_1-\overset{\oplus}{P}(Ph)_3\overset{\ominus}{Z} \quad (IV),$$

dans laquelle

Z représente un anion chlorure ou bromure, et

$Q_1$ représente un groupe $-(CH_2)_p$-phénylène-$(CH_2)_p$- ou $-(CH_2)_p$-cyclohexylidène-$(CH_2)_p$-, dans lequel p est un nombre pouvant valoir 1 ou 2, avec deux moles d'un ester d'acide carboxylique de formule V

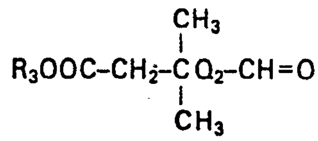

(V)

dans laquelle

$Q_2$ représente une liaison de valence ou un groupe $-CH_2-$, et

$R_3$ représente un groupe alkyle inférieur, on hydrogène les composés insaturés obtenus, on introduit, si on le souhaite, en position α et ω les substituants alkyle, alcoxy, halogéno ou hydroxyle, et, si on le souhaite, on transforme ensuite les esters, amides ou sels obtenus pour avoir des acides libres, ou bien on transforme les acides libres en des sel des esters ou amides.

7. Médicament, caractérisé en ce qu'il contient un ou des composés selon l'une des revendications 1 à 5 et des supports, excipients et adjuvants usuels.

8. Utilisation des composés selon l'une des revendications 1 à 5 pour préparer des médicaments ayant une action d'abaissement du taux des lipides et un effet antidiabète.

## Claims

1. α,ω-dicarboxylic acids having the general formula I'

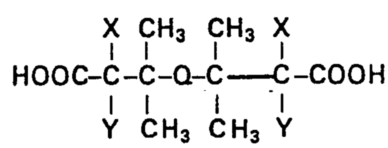

(I')

in which

X and Y, which can be the same or different, denote hydrogen, $C_1$–$C_6$-alkyl, $C_1$–$C_6$-alkoxy, hydroxy, cyano, halogen, carboxyl, $C_1$–$C_6$-alkoxycarbonyl or carbamoyl, and

Q denotes a $-(CH_2)_n$-cyclohexylidene-$(CH_2)_n$ group or a $-(CH_2)_m$-phenylene-$(CH_2)_m$ group or a $-CH_2-CH=CH$-phenylene-$CH=CH-CH_2$ or a $-CH_2-CH=CH-CH_2$-phenylene-$CH_2-CH=CH-CH_2$ group,

n denotes the numbers 2, 3 or 4 and

m is 3 or 4, and carboxylic acid derivatives thereof which can be hydrolysed in vivo.

2. Compounds according to claim 1, in which Y denotes hydrogen, X denotes hydrogen, halogen, methoxy, hydroxy, cyano, ethoxycarbonyl, carbamoyl or carboxyl and Q has the stated meaning.

3. Compounds according to claim 1 or 2, in which X and Y each denotes hydrogen and Q has the stated meaning.

4. 1–4-phenylene-bis-(3,3-dimethyl-6-yl hexanoic acid) and 1,4-phenylene-bis-(3,3-dimethyl-6-yl-5-hexenoic acid) according to any of claims 1, 2 or 3.

5. Compounds according to any of claims 1 to 4, in which carboxylic acids which can be hydrolysed in vivo denote salts with pharmacologically compatible alkyl metal or alkaline earth metal or ammonium bases, esters with low alcohols, amides with ammonia or lower alkyl amines or lactones if required.

6. A method of preparing α,ω-dicarboxylic acids having the general formula I'

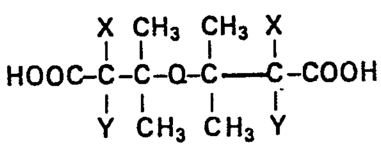

(I')

in which

X and Y, which can be the same or different, denote hydrogen, $C_1$–$C_6$-alkyl, $C_1$–$C_6$-alkoxy, hydroxy, cyano, halogen, carboxyl, $C_1$–$C_6$-alkoxycarbonyl or carbamoyl, and

Q denotes a $-(CH_2)_n$-cyclohexylidene-$(CH_2)_n$ group or a $-(CH_2)_m$-phenylene-$(CH_2)_m$ group or a $-CH_2-CH=CH$-phenylene-$CH=CH-CH_2$ or a $-CH_2-CH=CH-CH_2$-phenylene-$CH_2-CH=CH-CH_2$ group

n denotes the numbers 2, 3 or 4 and

m is 3 or 4, and carboxylic acid derivatives thereof which can be hydrolysed in vivo, characterised in that in known manner (a) a dihalide having the formula II

Hal–Q–Hal (II),

in which

Hal denotes chlorine or bromine and

Q has the stated meaning is converted into a bis-Grignard compound and the latter is reacted with 2 mols of a compound of formula III

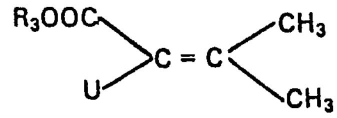

(III)

in which

$R_3$ denotes a lower alkyl group and

U denotes a $COOR_3$ group or a $-CONH_2$ or CN group, and the resulting compounds are saponified if required, decarboxylated if required and, if required, alkyl, alkoxy, hydroxy or halogen substituents are introduced in the α- and ω-position, or

b) in the case where X and Y denote hydrogen, halogen, hydroxy, alkyl or alkoxy, a bis-triphenyl phosphonium compound of formula IV

$$\overset{\ominus}{Z}(Ph)_3\overset{\oplus}{P}-Q_1-\overset{\oplus}{P}(Ph)_3\overset{\ominus}{Z} \quad (IV)$$

in which

Z denotes a chloride or bromide and

$Q_1$ denotes a $-(CH_2)_p$-phenylene-$(CH_2)_p$ group or

a $(CH_2)_p$-cyclohexylidene-$(CH_2)_p$ group in which p can be the number 1 or 2, is reacted with 2 mols of a carboxylic acid ester of formula V

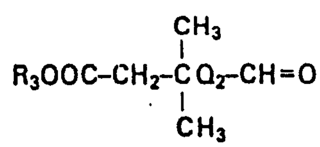

(V)

in which

$Q_2$ denotes a valency or a $-CH_2$ group and

$R_3$ denotes a lower alkyl group, the resulting unsaturated compounds are hydrogenated, alkyl, alkoxy, halogen or hydroxy substituents are introduced if required in the α- and ω position, and if required the resulting esters, amides or salts are subsequently converted into free acids or the free acids into salts, esters or amides.

7. A drug characterised by a content of compounds from any of claims 1 to 5 and conventional excipients and adjuvants.

8. Use of the compounds according to any of claims 1 to 5 for producing drugs having a lipid-lowering and anti-diabetic effect.